# EUROPEAN PATENT SPECIFICATION

(11) **EP 1 528 914 B1**
(45) Date of publication and mention of the grant of the patent: **01.10.2008**
(21) Application number: 03787793.3
(22) Date of filing: 12.08.2003
(51) Int. Cl.: A61K 9/00, A61K 9/19, A61K 47/26, A61K 39/00

(54) **ANTIGENIC COMPOSITIONS**
ANTIGENE ZUSAMMENSETZUNGEN
COMPOSITIONS ANTIGÉNIQUES

(30) Priority: 13.08.2002 GB 0218821
(43) Date of publication of application: 11.05.2005
(73) Proprietor: GlaxoSmithKline Biologicals s.a., 1330 Rixensart (BE)
(72) Inventor: VANDE VELDE, V. GlaxoSmithKline Biologicals S.A., B-1330 Rixensart (BE)
(74) Representative: Dalton, Marcus Jonathan William
(86) International application number: PCT/EP2003/009010
(87) International publication number: WO 2004/016241

(56) References cited:
- WO-A-86/03413
- WO-A-88/09674
- WO-A-96/32964
- FR-A- 1 594 506

## Description

The present invention relates to novel antigenic compositions suitable for parenteral and mucosal administration, comprising an antigen within an aqueous medium and a carbohydrate, wherein the carbohydrate is in the form of a derivative which exhibit acidic moieties, preferably in the form of its carboxylic acid derivative. The antigenic compositions are in a stable solid form and, after reconstitution with a liquid excipient or adjuvant followed by administration through the parenteral or mucosal route, rapidly dissolve in the body fluids, thereby releasing the reconstituted vaccine into the body. Specifically, the lyophilised form may consist of a powder or a cake containing the antigen, preferably in an amorphous (glassy) state, which is formed from a liquid solution or suspension by drying or sublimation, preferably sublimation by lyophilisation.

There is currently a great deal of interest in the pharmaceutical area in formulation that ensure the stability of the protein during the processing and storage stages. Aqueous liquid formulations are the easiest and more economical to handle during manufacturing, and are also the most convenient for the end-user. However chemical or physical degradations are commonly occurring phenomenon in liquid formulations, which lead to protein damages such as denaturation and degradation, often irreversible.

These problems can be overcome by using dried solids of stable protein mixtures, particularly but not only of stable lyophilised protein mixtures. Stable lyophilised formulations and the issues which may arise during their preparation have been described (Carpenter, J. 1997, Pharmaceutical Res. 14, 969-975). The formulation must be designed to ensure protein stability, to respect the desired product configuration, the product tonicity, especially for parenteral administration, and present an elegant cake structure. Similar considerations apply to other solid forms such as vacuum dried or spray-dried powders. Several requirements must be respected, an important one being to ensure isotonicity, critical for parenteral administration, especially when the product has a relatively low mass of protein per vial, as it is the case with vaccine formulations, which usually contain around 50 µg of protein per vial, on a total of around 15 mag. Salts, in particular NaCl, have been used to modify the tonicity of the mixture, but because it decreases the freezing point of solutions it can make the formulation more difficult to be frozen (due to a lower freezing point), and the elimination of water by sublimation will take more time. Polyalcohols and and carbohydrates such as mannitol or lactose have also been used with success to achieve a strong cake while respecting the isotonicity of the mixture (M.C. Lai and E.M. Topp, J. of Pharm. Sci., 1999, 88, 489-500).

WO 88 09 674 A discloses lyophilized compositions comprising gamma interferons and lactobionic acid; FR A 1 594 506 discloses lyophilised composition comprising an antiviral antigen and calcium lactobionate.

One important problem arises when the lyophilisation process must be carried out on mixtures which contain a high salt concentration as it is the case, for example, for proteins resulting from a purification process carried out in the presence of high salt concentrations. Alternatively, in some cases proteins may need a high salt concentration in order to remain in solution and/or to avoid aggregation and precipitation. These problems are particularly of issue when the salt is NaCl, since NaCl when present in solution gives a relatively low eutectic melting and make a formulation difficult to lyophilise properly.

Several solutions have been proposed, but these are unsatisfactory. The increase in the carbohydrate concentrations is associated with i) an increase in the osmotic pressure; ii) an incompatibility with parenteral administration, and iii) problems with the reconstitution itself, for example when the reconstitution liquid is already iso-osmotic by itself. Another alternative, the addition to the formulation of small quantities of mannitol, which cristallise easily and which leads to the formation of a sort of strong cristalline net in the cake (Kim, A. et al. J. Pharmaceutical Sc. 1998, 87, 931-935). does not apply to very high salt concentrations such as concentrations of NaCl above 15 mM and certainly about 30 mM. The addition to the formulation of a high molecular weight polymeric substance, unbiodegradable, as described by Taylor, L. and Zographi, G., J. Pharmaceutical Sc. 1998, 87, 1615-1621 may not compatible with pharmaceutical compositions or vaccine safety considerations.

The present invention offers an alternative to these formulations, which does not suffer from the disadvantages listed above. The present invention resides in the finding that the provision of antigenic compositions in a stable solid from is possible despite high salt concentrations being present which would have normally hampered the constitution of dried powders or of lyophilised cakes of acceptable structure. In particular, the present invention resides in the finding of suitable excipients termed bulking agents compatible with drying or lyophilisation of antigen mixtures containing high NaCl concentrations, in particular containing NaCl concentration above 30 mM. The excipients or bulking agents for use according to the present invention are advantageous since, through the ionic charges that they carry, they favour and even increase, in the presence of high NaCl concentrations, the intermolecular interactions between the bulking agent molecules, thereby allowing the cake formation to take place and to remain stable over time.

The invention further provides an antigenic composition in solid form such as lyophilised, spray-dried or spray freeze-dried or vacuum dried form and in reconstituted liquid form. According to the invention there is further provided a process for the preparation of a composition according to the invention which comprises mixing the ingredients of the composition, and either freezing them and drying the frozen mixture, or spraying them (for example into warm air), or vacuum drying them, or spray freezing-drying.

In one embodiment, the solid forms of the present invention are lyophilised porous forms, termed "cakes", or their ground form, which are stable during processing and storage, which are compatible with parenteral administration and which have a low residual moisture. The vaccine cakes of the present invention are formed from a liquid solution or suspension of vaccine by sublimation, and in a preferred form of the invention the sublimation is performed by lyophilisation.

In another embodiment the solid forms of the present invention are dried powders, particularly spray-dried or spray freezing-drying or vacuum-dried powders which have a low residual moisture.

Accordingly, it is an object of the invention to provide novel antigenic compositions suitable for freeze-drying, spray-drying or vacuum drying, comprising an antigen within an aqueous medium, a carbohydrate or derivative thereof which exhibit acidic moieties, and optionally a preservative. In a preferred embodiment, the aqueous antigenic medium contains a NaCl concentration of at least 15 mM, preferably of at least 30 mM. Typically the aqueous antigenic solution or suspension will be buffered, or pH adjusted to a pH compatible with parenteral or mucosal administration. Preferably the solid forms include at least one antigen in an amount from about 0.00001% to about 5% by weight. More preferably the antigen is in an amount of from about 0.001% to about 1% by weight. Still more preferably the antigen amount ranges from 0.01% to about 0.05%. Typically the NaCl concentration of the solution will range from about 15 mM to about 150 mM. More preferably the NaCl concentration will be at least 20 mM, most preferably at least about 30 mM. The bulking material will preferably be in an amount from about 50% to about 99.99999% by weight, more preferably from 60% to about 90%.

The bulking agent according to the invention provides both chemical and physical stability to the cake in the presence of high NaCl concentration and also prevents the cake to collapse. It also provides protection to the antigen. The bulking agent is a carbohydrate or derivative thereof, which is preferably in amorphous or cristalline state, preferebly in amorphous state. A preferred carbohydrate according to the invention concerns a carbohydrate or derivative thereof where high intermolecular interactions (as assessed for example by the melting point of the carbohydrates, the higher the melting point of the carbohydrate, the higher the intermolecular interactions in the crystal) can be obtained thereby allowing the solid form, in particular the cake structure, to be more resistant to the collapse phenomenon during the lyophilisation. Preferably, high molecular weight carbohydrates are used.

Carbohydrate derivatives are preferred which exhibit a reactive function, such as an amino moiety or an acidic moiety for example. Accordingly this is preferred aspect of the invention to use carbohydrate derivatives which exhibit acidic moieties, i.e. moieties which act as proton donors. The acidic moiety may be mono- or poly-acidic. Specific acidic moieties which may be mentioned include -O-P(O)(OH)₂ (phosphate ester), -O-P(O)-O-P(O)(OH)₂ (di phosphate ester), ), -O-P(O)-O-P(O)-O-P(O)(OH)₂ (triphosphate ester), -SO₄H (sulphate ester) and - CO₂H (carboxylic acid). Preferably the acidic moiety is carboxylic acid. The carbohydrate derivative may optionally be used in conjunction with a carbohydrate, such as sucrose for example.

The carbohydrate or carbohydrate derivatives may optionally be monohydrated, or polyhydrated (dihydrate, trihydrate, etc). In a still preferred embodiment, the carbohydrate is used in the form of its carboxylic acid derivative. Preferably, the carbohydrate is used in the form of the salt derivative of the carboxylic acid itself. Accordingly in one aspect the invention provides antigenic compositions suitable for freeze-drying, spray-drying spray freeze drying or vacuum drying, comprising an antigen within an aqueous medium, a carbohydrate, and optionally a preservative, wherein the carbohydrate is in the form of its carboxylic acid derivative.

Most preferably the carbohydrates derivatives are used in the form of their salt, preferably the calcium or sodium salt, most preferably their sodium salt.

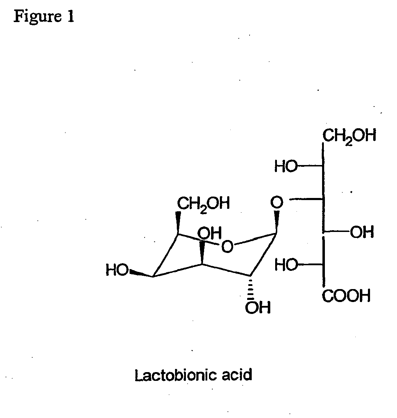

Non-limiting examples of suitable carbohydrates fulfilling the purposes of the invention include the carboxylic acid derivatives of the following: sucrose, maltose, trehalose, raffinose, lactose, fructose, galactose, mannose, maltulose, iso-maltulose and lactulose, or dextrose. Preferred bulking agents are the carboxylic acid derivatives of lactitol, sorbitol, glucose, galactose and galactitol, namely lactobionic acid, gluconic acid, glucuronic acid, galacturonic acid, and galactaric acid, respectively. These may be in their non hydrated or hydrated form (see Table 1). Even more preferably, lactobionic acid (pictured in Figure 1), glucuronic acid, gluconic acid and lactobionic acid are used, most preferably in a salt form favouring sugar-sugar intermolecular interactions. Sodium or calcium salts are preferred. The most preferred bulking agent is sodium lactobionate. Typically the carboxylic acid carbohydrate derivatives are used at a concentration (of the formulated liquid mix before lyophilisation) of about 80 mM and above. This provides an acceptable cake structure and ensure stability of the structre over time.

**Table 1. Carbohydrates and their carboxylic and salt derivatives**

| | | |
|---|---|---|
| Table 1 shows that in the crystallin solid state domain, molecule to molecule interactions are higher (as evidenced by a higher melting point) for ionic molecules compared to the corresponding non ionic molecules. | | |

| **Carbohydrate & derivatives** | **Characteristics** | **Melting points** |
|---|---|---|
| Lactitol | 4-O-β-D-galactopyranosyl-D-glucitol | 146°C |
| Lactobionic acid | 4-O-β-D-galactopyranosyl-D -gluconic acid A lactitol structure with one of the primary alcohol of the glucitol part replaced by a carboxylic acid; thus creating an opportunity for ionic bond interaction | 195°C |
| | | |
| Sorbitol or D-glucitol | 1,2,3,4,5,6-hexanehexol | 110°C |
| Gluconic acid | 2,3,4,5,6-pentahydroxy-hexanoic acid A glucitol structure with a primary alcohol replaced by a carboxylic acid; thus creating an ionic bond interaction | 131°C |
| Ammonium gluconat | The ammonium salt of gluconic acid | 154°C |
| | | |
| Glucose | α-D-glucopyranose | α: 146°C, β: 148°C |
| Glucuronic acid | A glucose with the primary alcohol replaced by a carboxylic acid; thus creating an ionic bond interactions | 165°C |
| | | |
| Galactose monohydrate | α-D-galactopyranose | 118°C |
| Galacturonic acid monhydrate | A galactose with the primary alcohol replaced by a carboxylic acid; thus creating an ionic bond interaction | α: 159°C, β: 166°C |
| | | |
| Galactitol | 1,2,3,4,5,6-hexanehexol | 188°C |
| Galactaric acid | A galactitol with two primary alcohols replaced by two carboxylic acids; thus creating two ionic bond interactions | 225°C |

By acceptable cake stucture is meant any, and preferably all of the following criteria:
- a cake volume similar to the volume of the starting liquid prior to the lyophilisation step;
- a cake having similar sizes measurement compared to the liquid prior to the lyophilisation step. In other words, a cake exibing as less retraction as possible in sizes measument compared to the initial liquid before the lyophilisation step;
- a cake showing visual homogeous aspect.

The cake size and volume measurement is made according to the procedure described in the Example section, under paragraph 3.2.

Furthermore, an acceptable cake structure is also understood to be a cake which does not show any change in its shape, size and color after having undergone an accelerated stability test during one week at 37°C, or during real time stability for a period of at least 6 months, preferably of at least one year, and ideally of at least two years.

The preservative is selected from antioxidants, free radicals scavengers and reducing agents. Preferred are antioxidants such as alkyl gallates, butylated hydroxyanisole, butylated hydroxytoluene, nordihydroguaiaretic acid and the tocopherols, ascorbic acid, isoascorbic acid, potassium and sodium salts of sulfurous acid, sodium formaldehyde sulphoxylate, citric acid, edetic acid and its salts, lecithin, tartaric acid, thiodiproionic acid.

The process for preparing the antigenic composition according to the present invention is suitably carried out using any lyophilisation, vacuum drying or spray drying, spray freezing drying technique commonly used within the pharmaceutical area. For example, the technique of lyophilisation, and details of other suitable excipients, may be found in Cameron et al., "Good Pharmaceutical freeze-drying Practice", Interpharm, Buffalo Grove (1997). Accordingly, in a further aspect the invention provides a process for preparing an antigenic composition as defined herein which comprises mixing the NaCl-comprising antigen solution, the bulking agent, and optionally the buffer and the preservative, and subjecting the mixture to a lyophilisation, vacuum drying or spray drying spray freezing drying procedure. A preferred process according to the invention is a vial freeze-drying process although ambient temperature evaporation is encompassed within the present invention. Such a process comprises aseptic filling, in sterile vials, of a sterile filtered solution of the antigenic composition, followed by sublimation by lyophilisation. A sterile freeze-drying rubber stopper is partially inserted into the vial, then the vial is frozen, e.g. at a temperature from -195.8°C to -5°C, preferably between - 80°C and -10°C, and thereafter vacuum-dried in the frozen state. After drying the stopper is fully inserted before removing the vial from the lyophilisation unit.

A preferred process according to the invention is a spray-drying process in which tiny droplets of the composition are obtained by pumping the liquid composition in a spray nozzel , with or without coaxial additional presurized air (or inert gaz). Such droplets being formed in a largelly ventillated area (at a temperature between +20°C and +250°C) so that quick drying by evaporation process is obtained. Dry powder is obtained after cyclone separation.

Another preferred process according to the invention is a spray freezing-drying process in which tiny droplets of the composition are obtained by pumping the liquid composition in a spray nozzle, with or without coaxial additional of presurized air (or inert gaz), such droplets being formed in a very cold chamber (between -5°C to -195.8°C) so that quick freezing of the droplet is obtained. Drying of this froozen material is obtained following the standard freeze drying process.

Another preferred process according to the invention is vaccum drying process. Such a process comprises aseptic filling of sterile vials with a sterile filtered solution of the composition. A sterile freeze-drying rubber stopper is partially inserted into the vial, then controlled vacumm is applied on the vial so evaporation proceed until final drying. After drying the stopper is fully inserted before removing the vial from the drying unit.

The lyophilisation or drying process is controlled so as to ensure a residual moisture level in the cake is below 3%, preferably below 2%, more preferably at around 1% or less. The antigen encapsulated therein will remain biologically active upon administration, that is to say capable of inducing an immune response in the host.

It is foreseen that compositions of the present invention will be used to formulate vaccines containing antigens derived from a wide variety of sources. For example, antigens may include human, bacterial, or viral nucleic acid, pathogen derived antigen or antigenic preparations, tumour derived antigen or antigenic preparations, host-derived antigens, including GnRH and IgE peptides, recombinantly produced protein or peptides, and chimeric fusion proteins.

Preferably the vaccine formulations of the present invention contain an antigen or antigenic composition capable of eliciting an immune response against a human pathogen, which antigen or antigenic composition is derived from HIV-1, (such as Tat, nef, gp120 or gp160), human herpes viruses, such as gD or derivatives thereof or Immediate Early protein such as ICP27 from HSV1 or HSV2, cytomegalovirus ((esp Human)(such as gB or derivatives thereof), Epstein Barr virus (such as gp350 or derivatives thereof), Varicella Zoster Virus (such as gpI, II and IE63), or from a hepatitis virus such as hepatitis B virus (for example Hepatitis B Surface antigen or a derivative thereof), hepatitis A virus, hepatitis C virus and hepatitis E virus, or from other viral pathogens, such as paramyxoviruses: Respiratory Syncytial virus (such as F and G proteins or derivatives thereof), parainfluenza virus, measles virus, mumps virus, human papilloma viruses (for example HPV6, 11, 16, 18, ..), flaviviruses (e.g. Yellow Fever Virus, Dengue Virus, Tick-borne encephalitis virus, Japanese Encephalitis Virus) or Influenza virus (whole live or inactivated virus, split influenza virus, grown in eggs or MDCK cells, or Vero cells or whole flu virosomes (as described by R. Gluck, Vaccine, 1992, 10, 915-920) or purified or recombinant proteins thereof, such as HA, NP, NA, or M proteins, or combinations thereof), or derived from bacterial pathogens such as *Neisseria spp,* including *N. gonorrhea* and *N. meningitidis* (for example capsular polysaccharides and conjugates thereof, transferrin-binding proteins, lactoferrin binding proteins, PilC, adhesins); *S. pyogenes* (for example M proteins or fragments thereof, C5A protease, lipoteichoic acids), *S. agalactiae, S. mutans, H. ducreyi; Moraxella spp, including M catarrhalis, also known as Branhamella* catarrhalis (for example high and low molecular weight adhesins and invasins); *Bordetella spp, including B. pertussis* (for example pertactin, pertussis toxin or derivatives thereof, filamenteous hemagglutinin, adenylate cyclase, fimbriae), *B. parapertussis and B. bronchiseptica; Mycobacterium spp., including M. tuberculosis* (for example ESAT6, Antigen 85A, -B or -C), *M. bovis, M. leprae, M. avium, M. paratuberculosis, M. smegmatis; Legionella spp,* including *L*. *pneumophila; Escherichia spp,* including *enterotoxic E. coli* (for example colonization factors, heat-labile toxin or derivatives thereof, heat-stable toxin or derivatives thereof), enterohemorragic *E*. *coli,* enteropathogenic *E*. *coli* (for example shiga toxin-like toxin or derivatives thereof); *Vibrio spp,* including *V. cholera* (for example cholera toxin or derivatives thereof); *Shigella spp,* including *S*. *sonnei, S. dysenteriae, S. flexnerii; Yersinia spp,* including *Y.* enterocolitica (for example a Yop protein) , *Y. pestis, Y. pseudotuberculosis; Campylobacter spp,* including *C. jejuni* (for example toxins, adhesins and invasins) and C. *coli; Salmonella spp,* including S. *typhi, S. paratyphi, S. choleraesuis, S. enteritidis; Listeria spp.,* including L. *monocytogenes; Helicobacter spp;* including *H.* pylori (for example urease, catalase, vacuolating toxin); *Pseudomonas spp,* including *P. aeruginosa; Staphylococcus spp.,* including *S*. *aureus, S. epidermidis; Enterococcus spp.,* including *E. faecalis, E. faecium; Clostridium spp.,* including C. tetani (for example tetanus toxin and derivative thereof), *C*. *botulinum* (for example botulinum toxin and derivative thereof), *C. difficile* (for example clostridium toxins A or B and derivatives thereof); *Bacillus spp.,* including *B. anthracis* (for example botulinum toxin and derivatives thereof); *Corynebacterium spp.,* (including *C*. *diphtheriae* (for example diphtheria toxin and derivatives thereof); *Borrelia spp.,* including *B. burgdorferi* (for example OspA, OspC, DbpA, DbpB), *B. garinii* (for example OspA, OspC, DbpA, DbpB), *B. afzelii* (for example OspA, OspC, DbpA, DbpB), *B. andersonii* (for example OspA, OspC, DbpA, DbpB), *B. hermsii; Ehrlichia spp.,* including *E*. *equi* and the agent of the Human Granulocytic Ehrlichiosis; *Rickettsia spp,* including *R. rickettsii; Chlamydia spp., including C. trachomatis* (for example MOMP, heparin-binding proteins), *C*. *pneumoniae* (for example MOMP, heparin-binding proteins), *C*. *psittaci; Leptospira spp.,* (including *L. interrogans; Treponema spp.,* including *T. pallidum* (for example the rare outer membrane proteins)*, T. denticola, T. hyodysenteriae;* or derived from parasites such as *Plasmodium spp.,* including *P. falciparum; Toxoplasma spp.,* including *T. gondii (for example SAG2, SAG3, Tg34); Entamoeba spp.,* including *E*. *histolytica; Babesia spp.,* including *B. microti; Trypanosoma spp.,* including *T. cruzi; Giardia spp.,* including *G. lamblia; Leshmania spp.,* including *L*. *major; Pneumocytis spp.,* including *P. carinii; Trichomonas spp.,* including *T*. *vaginalis; Schisostoma spp.,* including *S*. *mansoni,* or derived from yeast such as *Candida spp.,* including *C*. *albicans; Cryptococcus spp.,* including *C*. *neoformans.* In a preferred aspect of the invention, the rapidly dissolving vaccine cake for oral administration does not comprise rotavirus.

Preferred bacterial vaccines comprise antigens derived from *Streptococcus spp,* including *S*. *pneumoniae* (for example capsular polysaccharides and conjugates thereof, PsaA, PspA, streptolysin, choline-binding proteins)and the protein antigen Pneumolysin (Biochem Biophys Acta, 1989, 67, 1007; Rubins et al., Microbial Pathogenesis, 25, 337-342), and mutant detoxified derivatives thereof (WO 90/06951; WO 99/03884). Other preferred bacterial vaccines comprise antigens derived from *Haemophilus spp., including H. influenzae type B* (for example PRP and conjugates thereof), *non typeable H. influenzae,* for example OMP26, high molecular weight adhesins, P5, P6, protein D and lipoprotein D, and fimbrin and fimbrin derived peptides (US 5,843,464) or multiple copy varients or fusion proteins thereof. Other preferred bacterial vaccines comprise antigens derived from *Morexella Catarrhalis* (including outer membrane vesicles thereof, and OMP106 (WO97/41731)) and from *Neisseria mengitidis B* (including: outer membrane vesicles thereof, and NspA (WO 96/29412).

Derivatives of Hepatitis B Surface antigen are well known in the art and include, inter alia, those PreS1, PreS2 S antigens set forth described in European Patent applications EP-A-414 374; EP-A-0304 578, and EP 198-474. In one preferred aspect the vaccine formulation of the invention comprises the HIV-1 antigen, gp120, especially when expressed in CHO cells. In a further embodiment, the vaccine formulation of the invention comprises gD2t as hereinabove defined.

In a preferred embodiment of the present invention vaccines comprise an antigen derived from the Human Papilloma Virus (HPV) considered to be responsible for genital warts, (HPV 6 or HPV 11 and others), and the HPV viruses responsible for cervical cancer (HPV16, HPV18 and others).

Particularly preferred forms of genital wart prophylactic, or therapeutic, vaccine comprise L1 particles or capsomers, and fusion proteins comprising one or more antigens selected from the HPV 6 and HPV 11 proteins E6, E7, L1, and L2. The most preferred forms of fusion protein are: L2E7 as disclosed in WO 96/26277, and proteinD(1/3)-E7 disclosed in GB 9717953.5 (PCT/EP98/05285).

A preferred HPV cervical infection or cancer, prophylaxis or therapeutic vaccine, composition may comprise HPV 16 or 18 antigens. For example, L1 or L2 antigen monomers, or L1 or L2 antigens presented together as a virus like particle (VLP) or the L1 alone protein presented alone in a VLP or capsomer structure. Such antigens, virus like particles and capsomer are per se known. See for example WO94/00152, WO94/20137, WO94/05792, and WO93/02184. Additional early proteins may be included alone or as fusion proteins such as preferably E7, E2 or E5 for example; particularly preferred embodiments of this includes a VLP comprising L1E7 fusion proteins (WO 96/11272).

Particularly preferred HPV 16 antigens comprise the early proteins E6 or E7 in fusion with a protein D carrier to form Protein D - E6 or E7 fusions from HPV 16, or combinations thereof; or combinations of E6 or E7 with L2 (WO 96/26277). Alternatively the HPV 16 or 18 early proteins E6 and E7, may be presented in a single molecule, preferably a Protein D- E6/E7 fusion. Such vaccine may optionally contain either or both E6 and E7 proteins from HPV 18, preferably in the form of a Protein D - E6 or Protein D - E7 fusion protein or Protein D E6/E7 fusion protein.

The vaccine of the present invention may additionally comprise antigens from other HPV strains, preferably from strains HPV 6, 11, 31, 33, or 45.

Vaccines of the present invention further comprise antigens derived from parasites that cause Malaria. For example, preferred antigens from *Plasmodia falciparum* include RTS,S and TRAP. RTS is a hybrid protein comprising substantially all the C-terminal portion of the circumsporozoite (CS) protein of *P.falciparum* linked via four amino acids of the preS2 portion of Hepatitis B surface antigen to the surface (S) antigen of hepatitis B virus. It's full structure is disclosed in the International Patent Application No. PCT/EP92/02591, published under Number WO 93/10152 claiming priority from UK patent application No.9124390.7. When expressed in yeast RTS is produced as a lipoprotein particle, and when it is co-expressed with the S antigen from HBV it produces a mixed particle known as RTS,S. TRAP antigens are described in the International Patent Application No. PCT/GB89/00895, published under WO 90/01496. A preferred embodiment of the present invention is a Malaria vaccine wherein the antigenic preparation comprises a combination of the RTS,S and TRAP antigens. Other plasmodia antigens that are likely candidates to be components of a multistage Malaria vaccine are *P. faciparum* MSP1, AMA1, MSP3, EBA, GLURP, RAP1, RAP2, Sequestrin, PfEMP1, Pf332, LSA1, LSA3, STARP, SALSA, PfEXP1, Pfs25, Pfs28, PFS27/25, Pfs16, Pfs48/45, Pfs230 and their analogues in Plasmodium spp.

The formulations may also contain an anti-tumour antigen and be useful for the immunotherapeutic treatment cancers. For example, the adjuvant formulation finds utility with tumour rejection antigens such as those for prostrate, breast, colorectal, lung, pancreatic, renal or melanoma cancers. Exemplary antigens include MAGE 1 and MAGE 3 or other MAGE antigens for the treatment of melanoma, FRAME, BAGE or GAGE (Robbins and Kawakami, 1996, Current Opinions in Immunology 8, pps 628-636; Van den Eynde et al., International Journal of Clinical & Laboratory Research (submitted 1997); Correale et al. (1997), Journal of the National Cancer Institute 89, p293. Indeed these antigens are expressed in a wide range of tumour types such as melanoma, lung carcinoma, sarcoma and bladder carcinoma. Other Tumor-Specific antigens include, but are not restricted to Prostate specific antigen (PSA) or Her-2/neu, KSA (GA733), MUC-1, PRAME, carcinoembryonic antigen (CEA), PSCA (PNAS 95(4) 1735 -1740 1998), PSMA, P501S (sequence ID no 113 of Wo98/37814) also know as PS108 or prostein (WO 98/50567). Another tumour antigen is prostase, a prostate-specific serine protease (trypsin-like), disclosed in Ferguson, et al. (Proc. Natl. Acad. Sci. USA 1999, 96, 3114-3119) and in International Patent Applications No. WO 98/12302 (and also the corresponding granted patent US 5,955,306), WO 98/20117 (and also the corresponding granted patents US 5,840,871 and US 5,786,148) (prostate-specific kallikrein) and WO 00/04149 (P703P)Other prostate specific antigens are known from Wo98/37418, and WO/004149. Another antigen is STEAP PNAS 96 14523 14528 7 -12 1999. Other tumour associated antigens useful in the context of the present invention include: Plu -1 J Biol. Chem 274 (22) 15633 -15645, 1999, HASH-2 (WO 01/62778), Cripto (Salomon et al Bioessays 199, 21 61 -70, US patent 5654140) Criptin US patent 5 981 215. Additionally, antigens particularly relevant for vaccines in the therapy of cancer also comprise tyrosinase and survivin.

Other tumour-specific antigens are suitable for use in the present invention and include, but are not restricted to tumour-specific gangliosides such as GM 2, and GM3 or conjugates thereof to carrier proteins; or said antigen may be a self peptide hormone such as whole length Gonadotrophin hormone releasing hormone (GnRH, WO 95/20600), a short 10 amino acid long peptide, useful in the treatment of many cancers, or in immunocastration.

It is foreseen that compositions of the present invention will be used to formulate vaccines containing antigens derived from *Borrelia sp..* For example, antigens may include nucleic acid, pathogen derived antigen or antigenic preparations, recombinantly produced protein or peptides, and chimeric fusion proteins. In particular the antigen is OspA. The OspA may be a full mature protein in a lipidated form virtue of the host cell (E.Coli) termed (Lipo-OspA) or a non-lipidated derivative. Such non-lipidated derivatives include the non-lipidated NS1-OspA fusion protein which has the first 81 N-terminal amino acids of the non-structural protein (NS1) of the influenza virus, and the complete OspA protein, and another, MDP-OspA is a non-lipidated form of OspA carrying 3 additional N-terminal amino acids.

Vaccines of the present invention may be used for the prophylaxis or therapy of allergy. Such vaccines would comprise allergen specific (for example Der p1) and allergen non-specific antigens (for example peptides derived from human IgE, including but not restricted to the stanworth decapeptide (EP 0 477 231 B1)).

Upon use but before administration, the compositions according to the invention are generally reconstituted in a pharmaceutically acceptable diluent or carrier. Example of pharmaceutically acceptable diluent include water and saline. The reconstitution of the composition, or the vaccine, preferably takes place just before the administration of the composition to the patient. Preferably the time of dissolution of the cake is less than 10 seconds, more preferably less than 5 seconds, preferably less than 3 seconds and most preferably in less than 1 second.

In some embodiments of the present invention, the antigens will be formulated with a pharmaceutical carrier. Suitable pharmaceutical carriers for use in the vaccine according to the invention include those known in the art as being suitable for oral administration, especially to infants.

It may also be advantageous to formulate the virus of the invention in lipid-based vehicles such as virosomes or liposomes, in oil in water emulsions or with carrier particles.

Further immunostimulants which may advantageously be included are saponin derivatives such as QS21 and monophosphoryl lipid. A, in particular 3-de-O-acylated monophosphoryl lipid A (3D-MPL). Purified saponins as adjuvants are described in WO 98/56415. Saponins and monophosphoryl lipid A may be employed separately or in combination (e.g. WO 94/00153) and may be formulated in adjuvant systems together with other agents. 3D-MPL is a well-known adjuvant manufactured by Ribi Immunochem, Montana and its manufacture is described in GB 2122204.

Aluminium hydroxide is a particularly preferred component of a vaccine composition according to the invention.

Suitably a solution of the antigenic composition according to the invention and obtained after reconstitution is an isotonic solution. In a preferred embodiment the pH of the composition of the invention is from about 6 to about 10, preferably from about 7 to about 9. The antigenic composition of the invention when reconstituted is administered mucosally or parenterally, preferably parenterally, most preferably by injection intravenously, subcutaneously or intramuscularly. However, such formulations may be sought in another appropriate dosage form if necessary (e.g. nasal sprays and other mucosal forms).

The compositions according to the invention when for parenteral administration, may be packed in suitably adapted pharmaceutical application devices, for example syringes, vials or ampoules, such that the addition of the diluent or carrier allows in situ preparation of an aqueous solution of the active ingredient in a form suitable for immediate administration to the patient. Such devices form a further aspect of the invention. When for mucosal administration, lyophilised or dried formulations may conveniently be provided in the form of tablets in a pharmaceutical blister pack.

In another aspect the invention provides a composition comprising a live attenuated bacterium or virus, or live viral or bacterial vector, wherein the composition is a lyophilised solid or a dried solid according to the invention, capable of immediate dissolution when administered to the recipient.

Vaccines of the invention may be formulated and administered by known techniques, using a suitable amount of live virus to provide effective protection against infection without significant adverse side effects in typical vaccinees. A suitable amount of live virus will normally be between 10⁴ and 10⁷ ffu per dose. A typical dose of vaccine may comprise 10⁵-10⁶ ffu per dose and may be given in several doses over a period of time, for example in two doses given with a two-month interval. Benefits may however be obtained by having more than 2 doses, for example a 3 or 4 dose regimen, particularly in developing countries. The interval between doses may be more or less than two months long. An optimal amount of live virus for a single dose or for a multiple dose regimen, and optimal timing for the doses, can be ascertained by standard studies involving observation of antibody titres and other responses in subjects.

The amount of protein in each vaccine dose is selected as an amount which induces an immunoprotective response without significant, adverse side effects in typical vaccinees. Such amount will vary depending upon which specific immunogen is employed and how it is presented. Generally, it is expected that each dose will comprise 1-1000 µg of protein, preferably 1-500 µg, preferably 1-100µg, most preferably 1 to 50µg. An optimal amount for a particular vaccine can be ascertained by standard studies involving observation of appropriate immune responses in subjects. Following an initial vaccination, subjects may receive one or several booster immunisations adequately spaced.

The present invention is illustrated by the following examples.

### Example I

### Maximum PO₄-NaCl buffer concentration giving an acceptable lyophilised cake, using standard lyophilisation sugars

### 1.1. Introduction

Various phosphate (PO₄)/ NaCl buffer concentrations were submitted to a standard lyophilisation cycle.

The Phosphate - NaCl buffer was compared to a simple phosphate buffer (free of NaCl).

In those preparations, concentrations were adjusted in order to have comparable iso-osmotic pressure across each buffer for similar dilution (including osmotic pressure contribution of carbohydrate). In all tables presented hereafter, the concentration present in the final formulation just before the lyophilisation step is reported.

| **PO₄/NaCl** | | **PO₄** |
|---|---|---|
| 8/120 mM | | 120 mM |
| 6/90mM | | 90 mM |
| 4/60mM | | 60 mM |
| 2/30 mM | | 30 mM |
| 1/15 mM | | 15 mM |

### 1.2. Results

The aspect of the cake is assessed according to the criteria listed Example 3.2.

### 1.2.1. Sucrose (3.15%) compositions

| **PO₄ / NaCl** | **mOsm /Kg** | **cake at t = 0** | **cake at t = 1W at 37°C** | **cake t = 2W 37°C** | **Results** |
|---|---|---|---|---|---|
| 8-120mM | 297 | no cake | no cake | no cake | Fail |
| 6-90mM | 268 | no cake | no cake | no cake | Fail |
| 4-60mM | 204 | retracted | Retracted | Retracted | Fail |
| 2-30mM | 153 | slightly retracted | slightly retracted | slightly retracted | Pass |
| 1-15mM | 124 | slightly retracted | slightly retracted | slightly retracted | Pass |

| **PO₄** | **mOsm** | **cake** | **cake** | **cake** | **Results** |
|---|---|---|---|---|---|
| | **/Kg** | **at t = 0** | **at t = 1W at 37°C** | **t = 2W 37°C** | |
| 120mM | 352 | slightly retracted | retracted | retracted | Fail |
| 90mM | 299 | slightly retracted | slightly retracted | slightly retracted | Pass |
| 60mM | 218 | slightly retracted | slightly retracted | slightly retracted | Pass |
| 30mM | 160 | slightly retracted | slightly retracted | slightly retracted | Pass |
| 15mM | 127 | slightly retracted | slightly retracted | slightly retracted | Pass |

### 1.2.2. Maltose (3.15%) compositions

| **PO₄-NaCl** | **mOsm** | **cake** | **cake** | **cake** | **Results** |
|---|---|---|---|---|---|
| | **/ kg** | **T=0** | **T=1W37°C** | **T=2W37°C** | |
| 8-120mM | 299 | no cake | no cake | no cake | Fail |
| 6-90mM | 267 | retracted | retracted | retracted | Fail |
| 4-60mM | 206 | retracted | retracted | retracted | Fail |
| 2-30mM | 145 | slightly retracted | slightly retracted | slightly retracted | Pass |
| 1-15mum | 113 | slightly retracted | slightly retracted | slightly retracted | Pass |

| **PO₄** | **mOsm** | **cake** | **cake** | **cake** | **Results** |
|---|---|---|---|---|---|
| | **/ kg** | **T=0** | **T=1W37°C** | **T=2W37°C** | |
| 120mM | 334 | slightly retracted | retracted | retracted | Fail |
| 90mM | 272 | slightly retracted | retracted | retracted | Fail |
| 60mM | 215 | slightly retracted | slightly retracted | slightly retracted | Pass |
| 30mM | 153 | slightly retracted | slightly retracted | slightly retracted | Pass |
| 15mM | 117 | nice cake | slightly retracted | slightly retracted | Pass |

### 1.2.3. Trehalose (3.15%) compositions

| **PO₄-NaCl** | **mOsm** | **cake** | **cake** | **cake** | |
|---|---|---|---|---|---|
| | **/ kg** | **T=0** | **T=1W37°C** | **T=2W37°C** | **Results** |
| 8-120mM | 312 | no cake | no cake | no cake | Fail |
| 6-90mM | 265 | no cake | no cake | no cake | Fail |
| 4-60mM | 204 | retracted | retracted | retracted | Fail |
| 2-30mM | 142 | slightly retracted | slightly retracted | slightly retracted | Pass |
| 1-15mM | 112 | slightly retracted | slightly retracted | slightly retracted | Pass |

| **PO₄-Nacl** | **mOsm** | **cake** | **cake** | **cake** | |
|---|---|---|---|---|---|
| | **/ kg** | **T=0** | **T=1W37°C** | **T=2W37°C** | **Results** |
| 120mM | 326 | retracted | retracted | retracted | Fail |
| 90mM | 293 | retracted | retracted | retracted | Fail |
| 60mM | 215 | slightly retracted | slightly retracted | slightly retracted | Pass |
| 30mM | 146 | slightly retracted | slightly retracted | slightly retracted | Pass |
| 15mM | 111 | slightly retracted | slightly retracted | slightly retracted | Pass |

### 1.2.4. Raffinose (3.15%) compositions

| **PO₄-NaCl** | **mOsm** | **cake** | **cake** | **cake** | |
|---|---|---|---|---|---|
| | **/ kg** | **T=0** | **T=1W37°C** | **T=2W37°C** | **Results** |
| 8-120mM | 288 | no cake | no cake | no cake | Fail |
| 6-90mM | 232 | retracted | retracted | retracted | Fail |
| 4-60mM | 168 | slightly retracted | retracted | retracted | Fail |
| 2-30mM | 111 | slightly retracted | slightly retracted | slightly retracted | Pass |
| 1-15mM | 70 | slightly retracted | slightly retracted | slightly retracted | Pass |

| **PO₄** | **mOsm** | **cake** | **cake** | **cake** | |
|---|---|---|---|---|---|
| | **/ kg** | **T=0** | **T=1W37°C** | **T=2W37°C** | **Result** |
| 120mM | 289 | slightly retracted | slightly retracted | slightly retracted | Pass |
| 90mM | 231 | slightly retracted | slightly retracted | slightly retracted | Pass |
| 60mM | 173 | slightly retracted | slightly retracted | slightly retracted | Pass |
| 30mM | 118 | slightly retracted | slightly retracted | slightly retracted | Pass |
| 15mM | 80 | slightly retracted | slightly retracted | slightly retracted | Pass |

### 1. 2.5. Conclusions

The presence of Phosphate (PO₄ )/ NaCl in formulation is not compatible with lyophilisation. Indeed, the resulting cake structure is not acceptable when a concentration higher than 2 mM PO₄ / 30 mM NaCl is used. Sucrose, maltose, trehalose and raffinose give the same results when using the PO₄ / NaCl buffer.

NaCl is clearly responsible of the bad results during lyophilisation. Indeed, much higher concentrations can be reached with PO₄ (free of NaCl) buffer:
- up to 120 mM PO₄ with raffinose
- up to 90 mM PO₄ in sucrose
- up to 60 mM PO₄ for maltose and trehalose

### 1.3. Artistic view of sucrose molecular arrangement

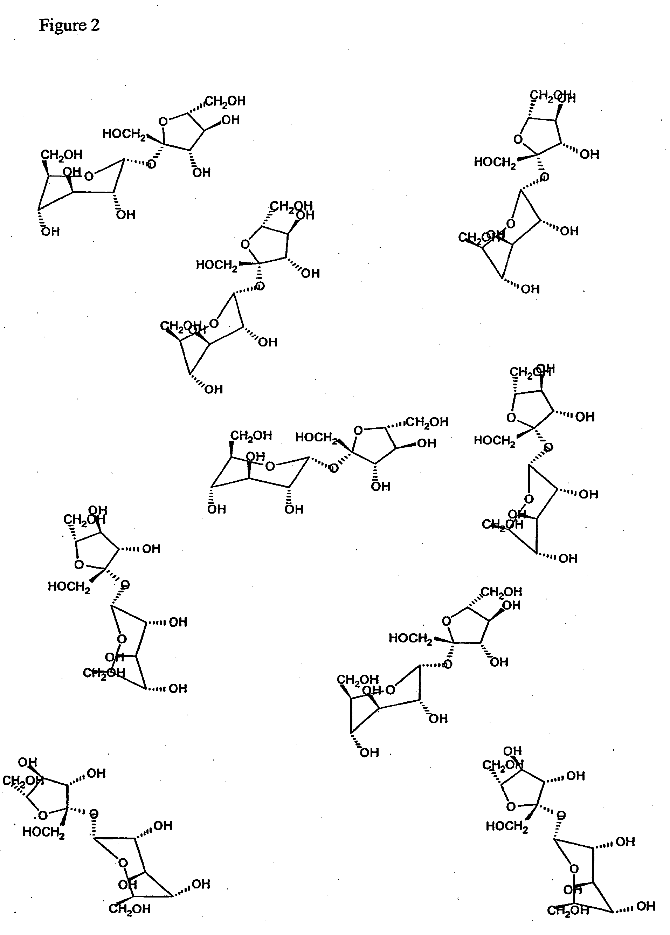

Figure 2 shows an artistic view of amorphous sucrose molecular arrangement in the absence of NaCl. The remaining water molecules are not shown.

The lyophilised cake structure is build essentially on the "sucrose-sucrose" molecular interactions. Those intermolecular interactions are the results of molecular orientation, molecular conformation and atomic radii of the constituent atoms.

Due to the amorphous structure (high degree of desorder between molecules), intermolecular interactions are weaker compared to those usually found in a crystalline structure (high degree of order between molecules).

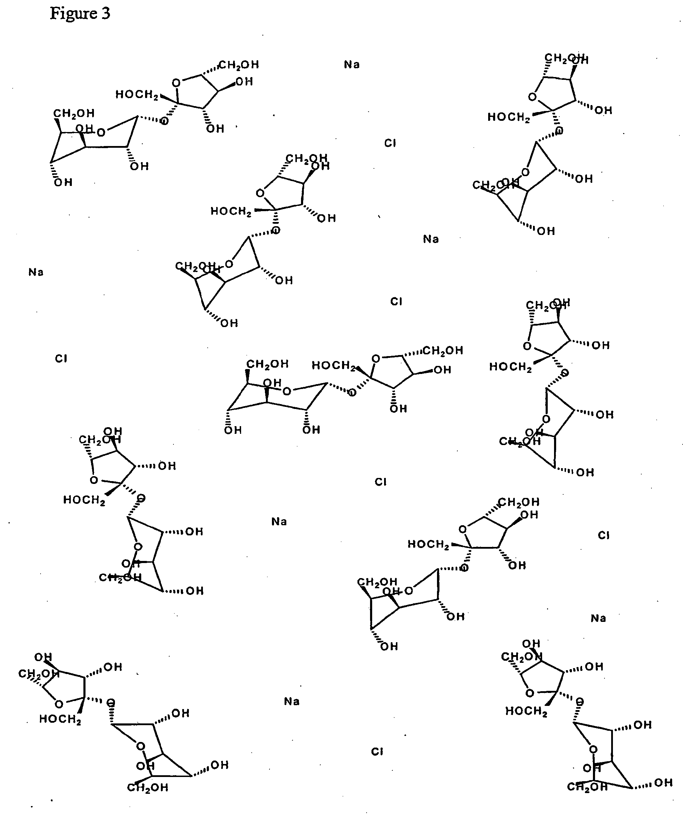

By comparison, Figure 3 shows an artistic view of amorphous molecular arrangement of about 90 mM sucrose in presence of 60 mM NaCl. The remaining water molecules are not shown.
The "sucrose-sucrose" molecular interactions are perturbed by the ionic interaction of sodium chloride. The experimental results shown in Example I that the presence of NaCl decreases the "sugar-sugar" intermolecular interactions.

### Example II

### Assessment of several carbohydrates (or excipients) leading to an acceptable lyophilised cake at a given fixed PO₄ (4mM) - NaCl (60mM) concentration

The target value for these experiments has been set at a concentration of PO₄ (6mM) - NaCl (60mM), a concentration of NaCl which, as shown in example I, leads to an unacceptable lyophilised cake structure, and which is often reached in a final antigenic solution after purification.

### Experiment 1

### Consolidation of the cake structure by using carbohydrate molecules exhibiting a carboxylic function, in addition to conventional sucrose.

Lactobionic acid is illustrated in Figure 1.

| **Sucrose** | **PO₄ /NaCl** | **Carbohydrate derivative** | **mM** | **Cake** | **Cake at 1 w 37°C** | **Result** |
|---|---|---|---|---|---|---|
| 2.08 % 4 mM /60 mM | | Ca- lactobionate | 9.6 mM | Slightly retracted | Slightly retracted | Pass |

### Experiment 2

Consolidation of the cake structure by using carbohydrate molecules exhibiting a carboxylic function, in the absence of sucrose.

Thus, in the lactobionate series, the nature of the salt (Na, Ca, or Tris counter-ion) plays a capital role in the "sugar-sugar" interactions. Tris molecule is bigger (compared to Na), and so is pushing "carbohydrate" molecules far away from each other, reducing the "sugar-sugar" interaction (compared to Na).

| | | | | | |
|---|---|---|---|---|---|
| At fixed NaCl concentrations: | | | | | |

| **PO₄** / NaCl | **Carbohydrate derivative** | **Carbohydrate mM** | **Cake at T = 0** | **Cake at 1W 37°C** | **Results** |
|---|---|---|---|---|---|
| 4mM / 60mM | Na lactobionate | 87 | OK | OK | Pass |
| 4mM / 60mM | Ca lactobionate | 87 | Slightly retracted | Slightly retracted | Pass |

| At decreasing NaCl concentrations: | | | | | | |
|---|---|---|---|---|---|---|
| **PO₄** / NaCl | **Carbohydrate derivative** | **Carbohydrate mM** | **MOs m kg** | **Cake at T = 0** | **Cake at 1 W 37°C** | **Result** |
| 4mM / 60mM | Na Lactobionate | 87 | 242 | Slightly retracted | Slightly retracted | Pass |
| 4mM / 60mM | Na Lactobionate | 80 | 251 | Slightly retracted | Slightly retracted | Pass |
| 0.8mM / 12mM | Na Lactobionate | 70 | 143 | Cake OK | Cake OK | Pass |
| 1.6mM / 24mM | Na Lactobionate | 70 | 175 | Slightly retracted | Slightly retracted | Pass |
| 2.4mM / 36mM | Na Lactobionate | 70 | 197 | Slightly retracted | Slightly retracted | Pass . |
| 3.2mM / 48mM | Na Lactobionate | 70 | 219 | Slightly retracted | Slightly retracted | Pass: limit |

### Experiment 3

### Consolidation of the cake structure by using carbohydrate molecules exhibiting a carboxylic function, using different carboxylic acid derivatives of carbohydrates.

| **PO₄/ NaCl** | **Carbohydrate derivative** | **Carbohydrate Na salt mM** | **mOs m/kg** | **Cake At T = 0** | **Cake At 7 W 37°C** | **Result** |
|---|---|---|---|---|---|---|
| 4mM / 60mM | Galacturonic | 90 mM | 272 | Slightly retracted | Slightly retracted | Pass |
| 4mM / 60mM | Galacturonic | 85 mM | 265 | Slightly retracted | Slightly retracted | Pass |
| 4mM / 60mM | Galacturonic | 80 mM | 254 | Slightly retracted | Slightly retracted | Limit |
| 4mM / 60mM | Galacturonic | 80 mM | 254 | Slightly retracted | Slightly retracted | Pass |
| 4mM / 60mM | Lactobionic | 100 mM | 280 | Slightly retracted | Slightly retracted | Pass |
| 4mM / 60mM | Lactobionic | 95 mM | 268 | Slightly retracted | Slightly retracted | Pass |
| 4mM / 60mM | Lactobionic | 90 mM | 263 | Slightly retracted | Slightly retracted | Limit |
| 4mM / 60mM | Na- Lactobionate | 92 mM | 277 | Slightly retracted | Slightly retracted | Pass |
| 4mM / 60mM | Na- Galacturonate | 92 mM | 247 | Slightly retracted | Slightly retracted | Pass |

### Example III

### Material and methods

### 1. Preparation of the antigenic composition

The formulated bulk mix is prepared by asepticly adding and mixing step by step successively the correct quantities of sterile water, sterile concentrated carbohydrate solutions, sterile concentrated buffer solutions, sterile concentrated salt solutions, and sterile concentrated antigen solutions.
The formulated bulk mix is distributed in the vials for lyophilisation (typically 3ml siliconed glass vials filled with 0.5ml of formulated bulk).

Lyophilisation rubber stoper is placed on vials (partially closed).

### 2. Lyophilisation

The vials containing (0.5ml) of formulated vaccine were placed inside a precooled (-69°C) lyophilisator (Martin-Christ or Usifroid), whilst the lyophilisation conditions were monitored and adjusted in order to follow as close as possible the following time-temperature curve: 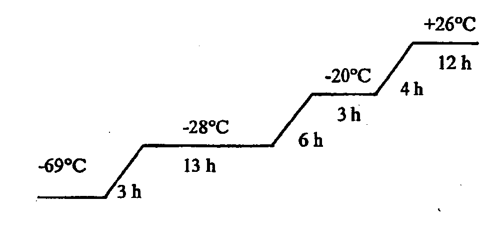 At the end of the processes under vacuum, the vials were closed by pushing the stopper completely.
Then stopper are sealed by aluminium capsules.
Lyophilised vials are storred at at +4°C until analysis or utilisation.

### 3. Inspection of the cake aspect

### 3.1. Accelerated stability at 37°C

Ten lyophilised vials of each formulation are placed for a period of one week in an ventilated oven regulated at 37°C, then stored at +4°C. The cake is then inspected.

### 3.2. Cake inspection

Lyophilised cakes are inspected visually just after lyophilisation and after accelerated stability tesing (7 days at 37°C).
The percentage of retraction is estimated by size measurement of the cakes, and classified according to one of the different categories:
- **Retracted** means that initial size of the cake (height or diameter) is reduced at least by 20%
- **Slightly retracted** means that the initial size of the cake (height or diameter) is reduced by a factor of at most 20%
- **Pass** means that the initial size of the cake (height or diameter) is reduced not more that 20% just after lyophilisation and also after 1 week at 37°C and also that not color change occurred after 1 week at 37°C
- **Fail** means that the initial size of the cake (height or diameter) is reduced at least by 20% just after lyophilisation or that after 1 week at 37°C or that a color change is ccurred after 1 week at 37°C

Measurement of the cake side is done applying a graduated rular on the bottom of the vials and measuring the cake diametre (or height) through the glass.

### 4. Buffers

Phosphate-NaCl buffer also known in house as the "Phosphate 10-150" is chosen because most of antigens are purified in this buffer. It should be pointed out that we used the NaH₂PO₄ + K₂HPO₄ combination. The selected pH value is obtained by weighting the correct NaH₂PO₄ / K₂HPO₄ proportion. Thus, no HCl or NaOH is used to adjust the pH of the buffer.
Decreasing quantities: 0.4, 0.3, 0.2, 0.1 and 0.05 ml of buffer are used during formulation. Final filling volume being always 0.5ml.

Phosphate buffer free of NaCl is chosen to be compared to the phosphate-NaCl buffer. Starting with a 150mM solution, decreasing quantities: 0.4, 0.3, 0.2, 0.1 and 0.05 ml of buffer are used during formulation.

### Osmotic pressure

Measurements were performed on a Friske Type one-ten osmometer.

## Claims

1. An antigenic composition suitable for freeze-drying, spray-drying, spray freezing-drying or vacuum drying comprising an antigen within an aqueous medium, a carbohydrate, and optionally a preservative, wherein the carbohydrate is in the form of a derivative which exhibit acidic moieties and wherein the aqueous medium comprises a NaCl concentration of at least 30 mM.

2. An antigenic composition as claimed in 1, wherein the carbohydrate is in the form of its carboxylic acid derivative.

3. An antigenic composition as claimed in 2, wherein the carboxylic derivative is selected from the list consisting of lactobionic acid, gluconic acid, glucuronic acid, galacturonic acid or galactaric acid.

4. An antigenic composition as claimed in any of claims 1 to 3 which is in spray-dried, vacuum-dried or freeze-dried form.

5. An antigenic composition as claimed in claim 4 which is in reconstituted form.

6. An antigenic composition as claimed in any one of claims 1 to 6, wherein the reconstituted composition comprises an adjuvant.

7. An antigenic composition as claimed in claim 6, wherein the adjuvant is selected from: oil-in-water emulsion, 3D-MPL, CpG, QS21 or a mixture of two or more thereof.

8. An antigenic composition as claimed in any one of claims 1 to 7, wherein the antigen or antigen composition is derived from the group comprising: Human Immunodeficiency Virus, Varicella Zoster virus, Hepres Simplex Virus type 1, Herpes Simplex virus type 2, Human cytomegalovirus, Dengue virus, Hepatitis A, B, C or E, Respiratory Syncytial virus, human papilloma virus, Influenza virus, Hib, Meningitis virus, Salmonella, Neisseria, Borrelia, Chlamydia, Bordetella, Plasmodium or Toxoplasma, stanworth decapeptide; or Tumour associated antigens (TMA), MAGE, BAGE, GAGE, MUC-1, Her-2 neu, LnRH, CEA, PSA, KSA, or PRAME, Cripto, HASH2, prostase, prostein.

9. A process for the preparation of an antigenic composition according to claims 1 to 3, comprising mixing the ingredients of the composition, and subjecting the mixture to a lyophilisation, vacuum drying or spray drying procedure:

10. A process for the preparation of an antigenic composition according to claim 1, comprising mixing the ingredients of the composition and either freezing them and drying the frozen mixture, or spraying them.

## Patentansprüche

1. Antigene Zusammensetzung, geeignet für Gefriertrocknen, Sprüh-Trocknen, Sprüh-Einfrieren-Trocknen oder Vakuum-Trocknen, die ein Antigen in einem wässrigen Medium, ein Kohlenhydrat und gegebenenfalls ein Konservierungsmittel umfasst, worin das Kohlenhydrat in der Form eines Derivates ist, das saure Reste aufweist, und worin das wässrige Medium eine NaCl-Konzentration von wenigstens 30 mM enthält.

2. Antigene Zusammensetzung gemäß Anspruch 1, worin das Kohlenhydrat in der Form seines Carbonsäure-Derivates ist.

3. Antigene Zusammensetzung gemäß Anspruch 2, worin das Carbonsäure-Derivat ausgewählt ist aus der Liste, die aus Lactobionsäure, Gluconsäure, Glucuronsäure, Galacturonsäure oder Galactarsäure besteht.

4. Antigene Zusammensetzung gemäß einem der Ansprüche 1 bis 3, die in einer sprühgetrockneten, vakuumgetrockneten oder gefriergetrockneten Form ist.

5. Antigene Zusammensetzung gemäß Anspruch 4, die in rekonstituierter Form ist.

6. Antigene Zusammensetzung gemäß einem der Ansprüche 1 bis 5, worin die rekonstituierte Zusammensetzung ein Adjuvans umfasst.

7. Antigene Zusammensetzung gemäß Anspruch 6, worin das Adjuvans ausgewählt ist aus: Öl-in-Wasser-Emulsion, 3D-MPL, CpG, QS21 oder einem Gemisch von zwei oder mehreren davon.

8. Antigene Zusammensetzung gemäß einem der Ansprüche 1 bis 7, worin das Antigen oder die Antigenzusammensetzung von der Gruppe stammt, die folgendes umfasst: humanes Immundefizienz-Virus, Varicella-Zoster-Virus, Herpes-Simplex-Virus Typ 1, Herpes-Simplex-Virus Typ 2, humanes Cytomegalievirus, Dengue-Virus, Hepatitis-A, -B, -C oder -E, respiratorisches Syncytialvirus, humanes Papillomavirus, Influenza-Virus, Hib, Meningitis-Virus, Salmonella, Neisseria, Borrelia, Chlamydia, Bordetella, Plasmodium oder Toxoplasma, Stanworth-Decapapeptid; oder Tumor-assoziierte Antigene (TMA), MAGE, BAGE, GAGE, MUC-1, Her-2 neu, LnRH, CEA, PSA, KSA oder PRAME, Cripto, HASH2, Prostase, Prostein.

9. Verfahren zur Herstellung einer antigenen Zusammensetzung gemäß den Ansprüchen 1 bis 3, das das Mischen der Bestandteile der Zusammensetzung und Unterziehen des Gemisches eines Lyophilisierungs-, Vakuum-Trocknungs- oder Spray-Trocknungsvorgangs umfasst.

10. Verfahren zur Herstellung einer antigenen Zusammensetzung gemäß Anspruch 1, das das Mischen der Bestandteile der Zusammensetzung und entweder Einfrieren dieser und Trocknen des gefrorenen Gemisches oder Sprühen dieser umfasst.

## Revendications

1. Composition antigénique adaptée pour la lyophilisation, la dessiccation par atomisation, la lyophilisation par atomisation ou la dessiccation sous vide, comprenant un antigène dans un milieu aqueux, un hydrate de carbone, et éventuellement un agent de conservation, dans laquelle l'hydrate de carbone est présent sous la forme d'un dérivé qui présente des fractions acides, et dans laquelle le milieu aqueux contient une concentration de NaCl d'au moins 30 mM.

2. Composition antigénique selon la revendication 1, dans laquelle l'hydrate de carbone est présent sous la forme de son dérivé d'acide carboxylique.

3. Composition antigénique selon la revendication 2, dans laquelle le dérivé carboxylique est choisi dans la liste comprenant l'acide lactobionique, l'acide gluconique, l'acide glucoronique, l'acide galacturonique ou l'acide galactarique.

4. Composition antigénique selon l'une quelconque des revendications 1 à 3, qui est présentée sous une forme desséchée par atomisation, desséchée sous vide ou lyophilisée.

5. Composition antigénique selon la revendication 4, qui est présentée sous une forme reconstituée.

6. Composition antigénique selon la revendication 5, dans laquelle la composition reconstituée comprend un adjuvant.

7. Composition antigénique selon la revendication 6, dans laquelle l'adjuvant est choisi parmi : une émulsion d'huile dans l'eau, 3D-MPL, CpG, QS21 ou un mélange de deux ou plus de ces derniers.

8. Composition antigénique selon l'une quelconque des revendications 1 à 7, dans laquelle l'antigène, ou la composition d'antigènes, est dérivé d'un groupe comprenant : le virus de l'immunodéficience humaine, le virus de la varicelle et du zona, le virus de l'herpès simplex de type 1, le virus de l'herpès simplex de type 2, le cytomégalovirus humain, le virus de la dengue, le virus de l'hépatite A, B, C, ou E, le virus respiratoire syncytial, le virus du papillome humain, le virus grippal, le Hib, le virus de la méningite, la salmonelle, Neisseria, Borrelia, Chlamydia, Bordetella, le plasmodium ou le toxoplasme, le décapeptide de stanworth ; ou des antigènes associés à une tumeur (TMA), MAGE, BAGE, GAGE, MUC-1, Her-2 neu, LnRH, CEA, PSA, KSA, ou PRAME, Cripto, HASH2, prostase, prostéine.

9. Procédé de préparation d'une composition antigénique selon les revendications 1 à 3, comprenant l'étape consistant à mélanger les ingrédients de la composition et l'étape consistant à soumettre le mélange à une procédure de lyophilisation, de dessiccation sous vide ou de dessiccation par atomisation.

10. Procédé de préparation d'une composition antigénique selon la revendication 1, comprenant l'étape consistant à mélanger les ingrédients de la composition et l'étape consistant soit à les congeler et à dessécher le mélange congelé, soit à les atomiser.
